# EUROPEAN PATENT APPLICATION

(11) **EP 1 464 358 A1**
(43) Date of publication of application: **06.10.2004**
(21) Application number: 04007402.3
(22) Date of filing: 26.03.2004
(51) Int. Cl.: A61M 25/01

(54) **Composite guidewire with a linear elastic distal portion**

(30) Priority: 31.03.2003 US 403848
(71) Applicant: Medtronic Interventional Vascular, Inc., Minneapolis MN 55432-5604 (US)
(72) Inventor: DeMello, Richard M., Acton Massachusetts 01720 (US); Flight, Bruce, Melrose Massachusetts 02176 (US)
(74) Representative: Hughes, Andrea Michelle

(57) **Abstract**

A composite guidewire 10 includes a central core 12 that is made out of a "linear elastic" material, which at body temperature does not exhibit a yield point and/or change phase when subjected to the range of stresses that are common to guidewires. A core extension 30, which is made out of stiffer material, such as stainless steel, attaches to the central core through a coupling tube, which may be made of super elastic material, linear elastic material, or stainless steel. The coupling tube 16 fits over a tapered distal end of the core extension and a proximal end of the central core that may but need not be tapered. The proximal end of the coupling tube abuts the tapered section of the core extension where the section has an outer diameter that is approximately the same size as the inner diameter of the coupling tube, and the tube is thus held against axial movement. The space between the inner diameter of the coupling tube and the ends that fit within the tube are filled with an adhesive, to create a strong yet flexible joint that transmits considerable torque without failure. An elongated coil 18, which may be radiopaque, fits over the distal portion of the central core. The coil may attach at a proximal end to the distal end of the coupling tube by, for example, adhesive, brazing or welding, such that the coil and the coupling tube provide support for the linear elastic core along the entire length of the core. The coil may instead extend over the coupling tube, such that the coil attaches to the distal end of the core extension. A shaping ribbon 20 which is made of bendable material, such as, for example, stainless steel, is attached to a distal portion of the central core and extends beyond the end of the core, to provide a shapeable distal end to the guidewire. An atraumatic tip 26 fits over the end of the guidewire and attaches to the coil and the shaping ribbon by brazing, welding or adhesive, to provide a cushioned end to the guidewire. As appropriate, a relatively short radiopaque outer coil may be positioned over the elongated coil, to provide visibility to the distal end of the guidewire.

## Description

### BACKGROUND OF THE INVENTION

### Field of the Invention

The invention relates generally to guidewires for directing catheters or other medical instruments through the cardiovascular system.

### Background Information

Guidewires for use in, for example, percutaneous transluminal coronary artery angioplasty (PTCA), must be thin and flexible enough to advance through small arteries within the coronary vasculature. These wires must also be sturdy enough to be manipulated from the outside of the body, such that a distal end of the wire can be brought into contact with a selected region of the coronary artery. Further, they must be strong enough to survive a "pull test" without breaking, to ensure that they do not come apart in the body.

Numerous guidewire designs exist. These designs have typically been made from stainless steel materials and may have platinum coils added to increase radiopacity. Coatings such as PTFE, silicon, and hydrophilic materials may be added to reduce friction and improve movement of devices that are passed over the guidewire.

Stainless steel guidewires are inherently stiff and offer excellent support along the proximal shaft portion of the guidewire. The distal ends of these wires may also be deliberately bent or shaped to aid in steering the guidewire into a particular vessel or lumen. The material, however, is susceptible to further plastic deformation during use and has been known to permanently deform and kink. The deformation is particularly noticeable when the guidewire is manipulated through a tortuous anatomy.

More recently, guidewires have been made from "super elastic" materials such as Nitinol, with coils added for radiopacity and coatings for lubricity, as mentioned above. The super elastic guidewires offer excellent kink resistance, and provide exceptional torque control when placed within tortuous anatomies. The super elastic material, however, is significantly less stiff than stainless steel and therefore does not provide a high level of support along the proximal portion of the guidewire.

Known prior composite construction guidewires combine a proximal portion of stainless steel with a distal portion of super elastic material, to take advantage of the best performance characteristics of both materials. Unfortunately, it is very difficult to attach non-super elastic materials to super elastic materials. The joint cannot, for example, be held together by conventional braising or welding. Accordingly, a special coupling must be used to lock the materials together.

In a known prior system the ends of the two guidewire portions, that is, the ends of the two materials, are butted against one another and a sleeve, which made of non-super elastic material, is fit over the joint. The guidewire portions are then held together by crimping, spot welding or gluing the sleeve in place. The coupling relies mainly on the mechanical interface between the two portions of the guidewire. If a mismatch in the cross-sectional dimensions exists, the distal and proximal portions may separate. Further, the repeated torqueing and bending that occurs when the guidewire is manipulated through the cardiovascular system may fatigue the coupling and result in the separation of the distal and proximal portions of the guidewire within the patient's body.

Another disadvantage of the super elastic material is that at body temperature the material undergoes a phase change to a low tensile strength martensite phase when the material is subjected to stress above a certain level. Accordingly, unless the system alters the temperature of the super elastic distal portion of the guidewire, the distal portion may not offer a sufficient level of support for the devices passed over the guidewire.

### SUMMARY OF THE INVENTION

The invention provides a composite guidewire as defined in claim 1. The composite guidewire constructed in accordance with the invention includes a central core or core section that is made out of a "linear elastic" material, preferably one which at body temperature does not exhibit a yield point and/or change phase when subjected to the range of stresses that are common to guidewires. The linear elastic material thus maintains an overall stiffness that is greater than the stiffness of the super elastic material used in known prior composite guidewires. In particular, the linear elastic material maintains a greater stiffness at and above the stress level at which the super elastic material yields and/or undergoes a change to a lower tensile strength phase. Accordingly, the linear elastic core provides greater support for the devices that are passed over the guidewire, while also providing kink resistance that is similar to that of the super elastic material.

A core extension, which is made out of stiffer material, such as stainless steel, preferably, attaches to the central core through an appropriately sized coupling tube, which may be made of super elastic material, linear elastic material, or stainless steel. The coupling tube preferably extends over a tapered distal end of the core extension and a tapered proximal end of the central core. The ends of the coupling tube preferably abut the portions of the respective tapering sections that have diameters that are approximately the same size as the inner diameter of the coupling tube. The coupling tube is thus held against axial movement. The space between the inner diameter of the coupling tube and the core and core extension ends that fit within the coupling tube is preferably filled with an adhesive, to create a strong yet flexible joint that is able to transmit considerable torque without failure. Further, the coupling tube provides support for the proximal end of the core.

The coil, which may be radiopaque, fits over the distal portion of the central core. The coil may attach at a proximal end to the distal end of the coupling tube by, for example, adhesive, brazing or welding, such that the coil and the coupling tube provide support for the linear elastic core along the entire length of the core. The shaping ribbon which is made of bendable material, such as, for example, stainless steel, is attached to a distal portion of the central core and extends beyond the end of the core, to provide a shapeable distal end to the guidewire. The tip is preferably an atraumatic tip which fits over the end of the guidewire and attaches to the coil and the shaping ribbon by brazing, welding or adhesive, to provide a cushioned end to the guidewire. As appropriate, a relatively short radiopaque outer coil may be positioned over the elongated coil, to provide visibility to the distal end of the guidewire.

The joint between the core and the core extension may instead by formed using a stainless steel coupling coil. The coupling coil is filled with adhesive to hold the tapered ends of the core and the core extension in the same manner as the coupling tube.

Preferred embodiments will now be described by way of example only, with reference to the drawings.

### BRIEF DESCRIPTION OF THE DRAWINGS

The invention description below refers to the accompanying drawings, of which:
FIG. 1 is a cross-sectional view of a guidewire constructed in accordance with the invention;
FIG. 2 depicts the guidewire of FIG. 1 with a radiopaque cover;
FIG. 3 depicts the guidewire of FIG. 1 with a core extension;
FIG. 4 depicts the guidewire of FIG. 3 with an alternative coil and coupling tube arrangement.
FIG. 5 depicts the guidewire of FIG. 3 with an alternative coupling arrangement.
FIG. 6 depicts an alternative to the guidewire of FIG. 3; and
FIG. 7 depicts the guidewire of FIGS. 3 or 4 with a coupling coil.

### DETAILED DESCRIPTION OF AN ILLUSTRATIVE EMBODIMENT

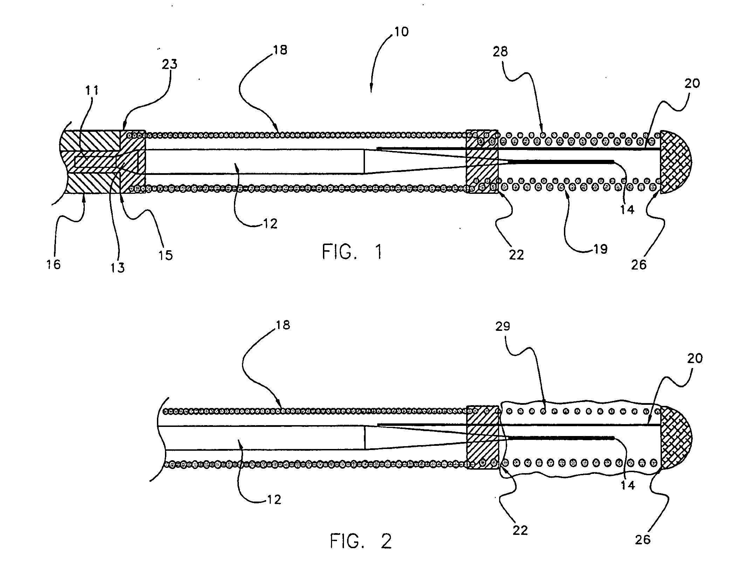

Referring now to FIG. 1, a composite guidewire 10 includes a central core 12 which is made of a "linear elastic" Nitinol material. The linear elastic material exhibits an essentially linear stress-strain function at body temperature. The linear elastic material thus does not change to a lower tensile strength phase when the material is subjected to the stresses associated with guidewire advancement through the body. Accordingly, the linear elastic material is stiffer than the super elastic Nitinol material at stress levels that exceed the level associated with the phase change in the super elastic material. The linear elastic material thus provides a relatively high level of support for the distal end of the guidewire when the guidewire is in place in the body, without requiring a change in the temperature of the material.

A coupling tube 16 fits over a tapered proximal end 11 of the central core 12, with the distal end 15 of the tube abutting a tapered core section 13. The end of the tube abuts the tapered section where the section has an outer diameter that meets or exceeds the inner diameter of the tube. The coupling tube 16 is filled with an adhesive, such as an epoxy resin, and the tube is then held in place on the proximal end of the core. The coupling tube may be made of super elastic material, linear elastic material, stainless steel, or other flexible material.

A shaping ribbon 20 attaches to a distal portion of the central core 12 and extends beyond the distal end 14 of the central core 12, to provide a shapeable end. The distal end of the central core may be tapered (as shown), to provide additional flexibility to the distal end of the wire. Further, the windings 19 of a coil 18 may be slightly spread apart to provide even greater flexibility.

An elongated coil 18 extends from a distal end of the guidewire along the central core 12 and attaches to the distal end 15 of the coupling tube 16. The coil 18 and the coupling tube attach to one another at their respective ends by, for example, soldering, brazing or adhesive as indicated by reference numeral 23. The coil also attaches at its distal end to the distal end of the shaping ribbon 20. The coil and the ribbon may also attach to one another by, for example, soldering, at one or more locations 22 along their lengths.

An atraumatic tip 26 attaches to the distal ends of the coil 18 and the ribbon 20, to provide a cushioned distal end to the guidewire 100.

The elongated coil 18 is preferably non-radiopaque, and a shorter radiopaque coil 28 fits over the coil 18 to provide visibility under x-ray. As shown, the coil 18 may be tapered at its distal end and the radiopaque coil 28 positioned over the tapered section of the coil 18, such that the guidewire has a uniform outer diameter along the length of the central core.

Alternatively, the coil 18, or a portion thereof, may be radiopaque and the coil 28 may be omitted.

Referring now to FIG. 2, the radiopacity may instead be provided by a radiopaque plastic cover 29 that fits over the distal end of the wire and is attached to the coil 18 by, for example, heat shrinking. The cover 29 may fit over the tip 26 or may incorporate a cushion (not shown) and thus replace the tip.

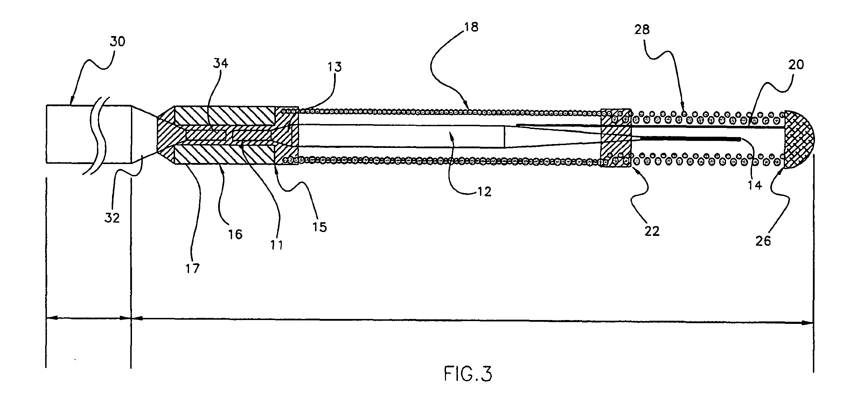

Referring now to FIG. 3, the guidewire 10 further includes a core extension 30 that is made of a stiffer material, such as, stainless steel. The core extension operatively joins to the central core 12 through the coupling tube 16. A distal end 32 of the core extension tapers to a tapered end section 34 that fits within the coupling tube, such that the outer diameter of the guidewire remains constant from a proximal end 17 of the coupling tube to the distal end of the coils 18 and 28.

The proximal end 17 of the coupling tube 16 abuts the tapered section 32 of the core extension, where the outer diameter of the section is approximately same as the inner diameter of the coupling tube. The tapered sections 32 and 13 of the core extension and the central core hold the coupling tube 16 against axial movement. As discussed above, the coupling tube 16 is filled with an adhesive, in order to provide a strong and relatively flexible joint between the core and the core extension.

The distal portion of the guidewire, up to and including the coupling tube 16 and the tapered section 32 of the core extension 30 are preferably coated with a hydrophilic coating. The remainder of the guidewire is coated with a PTFE coating.

The coupling tube 16 is approximately ten centimeters in length. The respective ends 11 and 34 the core and the core extension extend approximately five centimeters within the tube, and thus provide relatively large bonding areas for the joint. In addition to providing a strong yet flexible joint, the relatively long coupling tube also provides support for the proximal end of the central core.

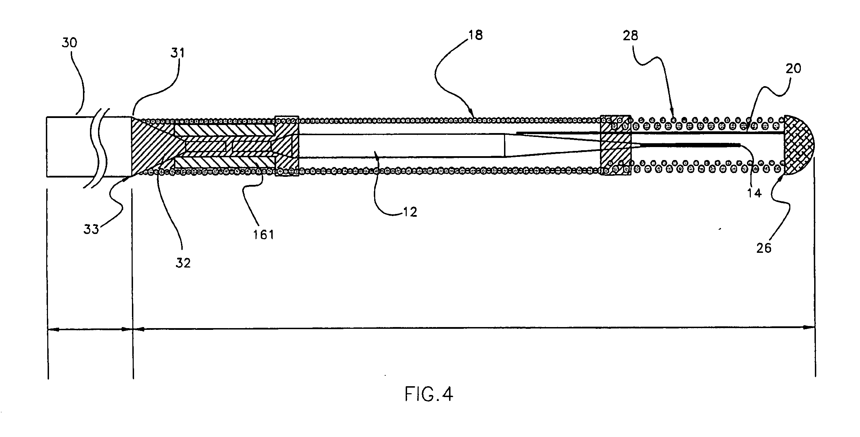

Referring now to FIG. 4, a coupling tube 161 is provided that has a smaller outer diameter than the coupling tube 16, and the coil 18 extends over the coupling tube 161 to provide additional support for the proximal end of the core 12. The lengthened coil 18 attaches at its proximal end to the proximal end 31 of the tapered section 32 of the core extension 30, such that the guidewire has a uniform outer diameter essentially from the distal end of the guidewire to the proximal end of the guidewire. As indicated by reference numeral 33, the coil 18 attaches to the core extension by adhesive, soldering, brazing, welding or other conventional attachment techniques. Further, an adhesive may be applied over the tapered section 32, to attach the coil also to the proximal end of the coupling tube 161.

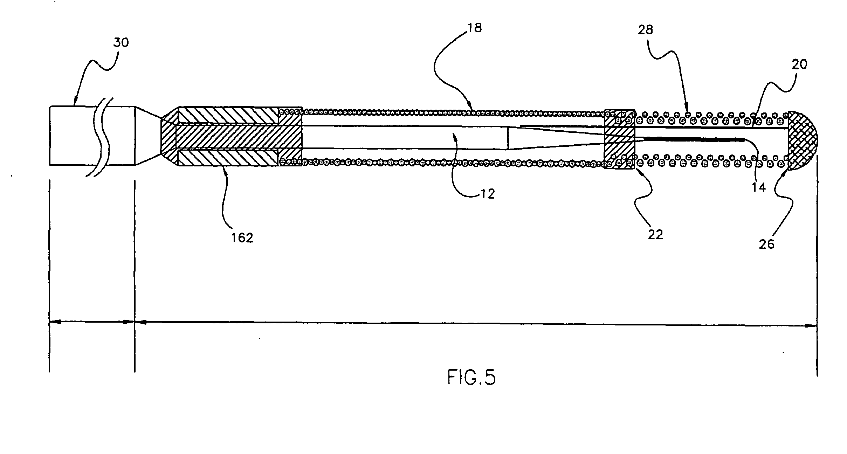

Referring to FIG. 5, the core may not taper at its proximal end, and instead fit directly into the coupling tube 162, which has a larger inner diameter than the coupling tube 16 discussed above. The coupling tube 162 then extends along the core and attaches to the portion of the non-tapering core to which the coil 18 extends. As discussed with reference to FIG. 3 above, the coil 18 attaches to the distal end of the coupling tube 16. The coupling arrangement depicted in FIG. 5 is preferably used with larger diameter guidewires, such as guidewires with 018 inch (0.4572 mm) outer diameters, that require less support at their proximal ends than the guidewires with smaller .14 inch (0.3556 mm) outer diameter.

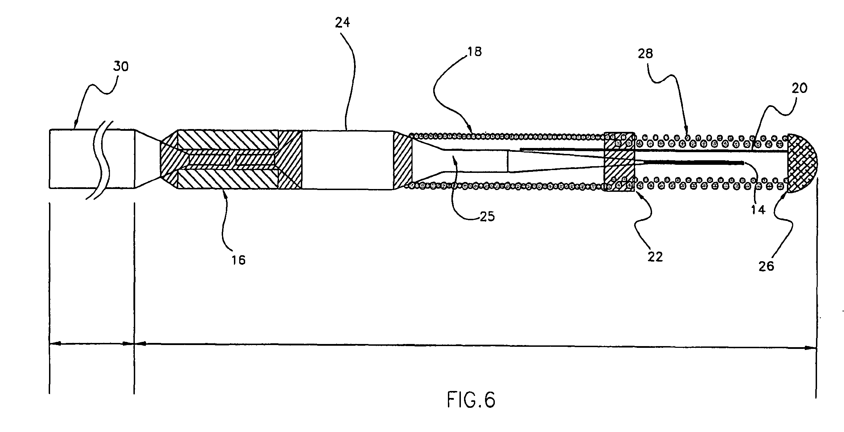

As shown, the proximal end of the coil 18 may extend to and connect with the distal end of the coupling tube and/or extend over the coupling tube, such that the linear elastic core is provided with support over its entire length. Alternatively, as depicted in FIG. 6, the linear elastic core may include a mid-section 24 that has the same outer diameter as the guidewire. The linear elastic material is sufficiently stiff that the thicker mid-section does not require the support of the coil 18, and the coil 18 then extends from the distal end of the guidewire to a tapered distal end 25 of the mid-section 24. The distal end 25 of the mid-section 24 tapers to a diameter that fits within the coil 18, such that the guidewire has a constant outer diameter from the coupling tube to the distal end of the shaping ribbon.

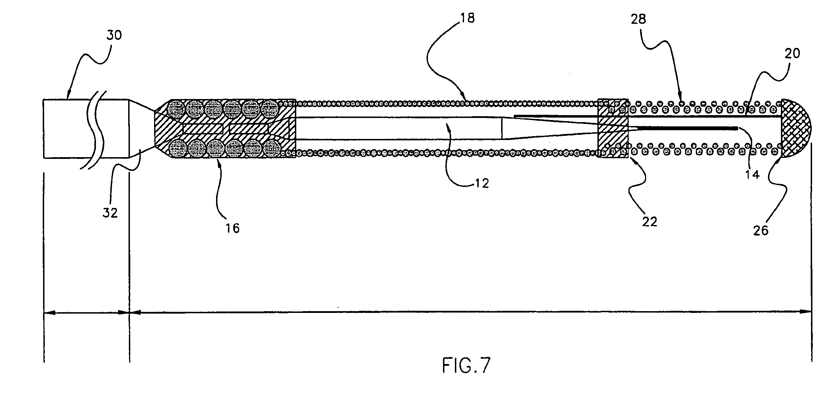

As depicted in FIG. 7 the coupling tube 16 may be replaced by a stainless steel coupling coil 160. The tapered end sections 11 and 34 of the core and the core extension extend into the coupling coil, and the coil is then filled with an adhesive, to provide a strong yet flexible joint. The joint between the core and the core extension may instead be formed using any type of flexible member that accepts the ends of the core and the core extension and retains them by adhesive or other known retention techniques. The member must, however, be strong enough to transmit the torque generated during guidewire use.

In summary, the composite guidewire includes a linear elastic central core and a stainless steel core extension that are connected via a coupling tube or other flexible coupling member that provides a strong yet flexible joint and also supports the proximal end of the linear elastic core. The linear elastic core provides flexibility and has sufficient stiffness at body temperature and under stress to also provide support for devices that pass over the distal section of the guidewire. The shaping ribbon and one or more coils provide a flexible and shapeable distal end. Accordingly, the guidewire with its stiffer, stainless steel core extension, its linear elastic central core and its shapeable distal end combines the best qualities of the various materials.

The foregoing description has been limited to specific embodiments of this invention. It will be apparent, however, that variations and modifications may be made to the invention, the scope of which is defined by the claims, including substituting other linear elastic and/or super or non-super elastic materials for those specifically named herein, using coupling members of various shapes and materials, using shaping ribbons of various cross-sectional shapes and materials, with the attainment of some or all of its advantages.

## Claims

1. A composite guidewire (10) including:
a core or core section (12) comprising linear elastic material, the core or core section having a distal portion and a proximal portion;
a coupling member (16) extending over the proximal portion (11) of the core or core section (12), the coupling member having a distal end (15) and a proximal end;
a shaping ribbon (20) of a shapeable material, the ribbon attaching to a distal portion of the core or core section and extending beyond the distal end of the core or core section,
a coil (18) engaged over the distal portion of the core or core section, the elongated coil having a distal end that attaches to the distal end of the shaping ribbon and a proximal end that attaches to the coupling member; and
a tip (26) attached to the distal ends of the coil and the shaping ribbon.

2. The composite guidewire of claim 1 further including a core extension (30) of a relatively stiff material, the core extension having a distal end that joins to a proximal end of the core or core section through the coupling member.

3. The composite guidewire of claim 2 wherein the proximal end of the coil (18) attaches to the distal end of the coupling member.

4. The composite guidewire of claim 3 wherein the core or core section and the core extension have tapered ends (13, 32) that fit with the coupling member and the space between the tapered ends and the inner diameter of the coupling member is filled with an adhesive.

5. The composite guidewire of claim 4 wherein the proximal end of the coupling member abuts the distal end of the core extension at the point where the end of the core extension tapers to approximately the size of the inner diameter of the coupling member.

6. The composite guidewire of claim 5 wherein the distal end of the coupling member abuts the proximal end of the core or core section at the point where the end of the core or core section tapers to approximately the size of the inner diameter of the coupling member.

7. The composite guidewire of claim 2 wherein the coil extends over the coupling member and attaches at the distal end to the core extension.

8. The composite guidewire of claim 7 wherein the core extension has a tapered distal end and the coil attaches to the core extension at a proximal end of the tapered section.

9. The guidewire of claim 1 wherein a distal end segment of the core or core section is tapered.

10. The guidewire of claim 9 wherein
the coil is non-radiopaque, and
the guidewire further includes a radiopaque coil (28) engaged over the non-radiopaque coil.

11. The guidewire of claim 9 wherein
the coil is non-radiopaque, and
the guidewire further includes a radiopaque cover (29) engaged over the tip and the distal end of the coil.

12. The guidewire of claim 1, further including:
a core extension of a relatively stiff material; and wherein the coupling member attaches to a tapered proximal section of the core or core section at a point where the cross-section has an outer diameter that corresponds to the inner diameter of the coupling member,
attaches to a tapered distal section of the core extension at a point where the cross-section has an outer diameter that corresponds to the inner diameter of the coupling member; and
is filled with an adhesive to retain the ends and provide a joint between the central core and the core extension.

13. The composite guidewire of any preceding claim wherein the linear elastic material is a Nitinol alloy that at body temperature remains in a single phase under the levels of stress associated with guidewire use.

14. The composite guidewire of any preceding claim wherein the coupling member is a flexible coupling tube made of linear elastic material, super elastic material, or stainless steel.

15. The composite guidewire of any of claims 1 to 13, wherein the coupling member is a flexible coupling coil made of stainless steel.
